# EUROPÄISCHE PATENTSCHRIFT

(11) **EP 2 542 567 B1**
(45) Veröffentlichungstag und Bekanntmachung des Hinweises auf die Patenterteilung: **12.02.2020**
(21) Anmeldenummer: 11707643.0
(22) Anmeldetag: 02.03.2011
(51) Int. Cl.: C07K 14/31, C07K 1/32

(54) **SELEKTIVE ANREICHERUNG VON ANTIKÖRPERN**
SELECTIVE ENRICHMENT OF ANTIBODIES
ENRICHISSEMENT SÉLECTIF D'ANTICORPS

(30) Priorität: 05.03.2010 EP 10155647
(43) Veröffentlichungstag der Anmeldung: 09.01.2013
(73) Patentinhaber: BOEHRINGER INGELHEIM INTERNATIONAL GMBH, 55216 Ingelheim am Rhein (DE)
(72) Erfinder: AMBROSIUS, Dorothee, 55216 Ingelheim Am Rhein (DE); DIETERLE, Michael, 55216 Ingelheim Am Rhein (DE); DOBBERTHIEN, Philine, 55216 Ingelheim Am Rhein (DE); HOYER, Maria-Katharina, 55216 Ingelheim Am Rhein (DE)
(74) Vertreter: Simon, Elke Anna Maria
(86) Internationale Anmeldenummer: PCT/EP2011/053126
(87) Internationale Veröffentlichungsnummer: WO 2011/107518

(56) Entgegenhaltungen:
- EP-A1- 2 157 099
- WO-A1-92/09633
- US-A1- 2007 072 307
- JING PING CHEN ET AL: "POLYMER-PROTEIN CONJUGATES. \II. AFFINITY PRECIPITATION SEPARATION OF HUMAN IMMUNOGAMMAGLOBULIN BY A POLY(N-ISOPROPYLACRYLAMIDE)-PROTEIN A CONJUGATE", BIOMATERIALS, ELSEVIER SCIENCE PUBLISHERS BV., BARKING, GB LNKD- DOI:10.1016/0142-9612(90)90020-Q, Bd. 11, Nr. 9, 1. November 1990 (1990-11-01), Seiten 631-634, XP000172543, ISSN: 0142-9612
- NILSSON B ET AL: "A synthetic IgG-binding domain based on staphylococcal protein A", PROTEIN ENGINEERING, OXFORD UNIVERSITY PRESS, SURREY, GB, Bd. 1, Nr. 2, 1. Januar 1987 (1987-01-01), Seiten 107-113, XP002133892, ISSN: 0269-2139
- LABROU N ET AL: "THE AFFINITY TECHNOLOGY IN DOWNSTREAM PROCESSING", JOURNAL OF BIOTECHNOLOGY, ELSEVIER SCIENCE PUBLISHERS, AMSTERDAM, NL LNKD- DOI:10.1016/0168-1656(94)90047-7, Bd. 36, Nr. 2, 15. August 1994 (1994-08-15), Seiten 95-119, XP000674449, ISSN: 0168-1656
- LJUNGQUIST C ET AL: "Thiol-directed immobilization of recombinant IgG-binding receptors", EUROPEAN JOURNAL OF BIOCHEMISTRY, WILEY-BLACKWELL PUBLISHING LTD, GB, vol. 186, no. 3, 22 December 1989 (1989-12-22), pages 557-561, XP002555465, ISSN: 0014-2956, DOI: 10.1111/J.1432-1033.1989.TB15244.X
- BOTTOMLEY S P ET AL: "Elution of human IgG from affinity columns containing immobilised variants of protein A", JOURNAL OF IMMUNOLOGICAL METHODS, ELSEVIER SCIENCE PUBLISHERS B.V.,AMSTERDAM, NL, vol. 182, no. 2, 9 June 1995 (1995-06-09), pages 185-192, XP004021167, ISSN: 0022-1759, DOI: 10.1016/0022-1759(95)00049-G

## Beschreibung

### HINTERGRUND DER ERFINDUNG

### TECHNISCHES GEBIET

Die vorliegende Erfindung betrifft Reinigungsverfahren für Proteine, insbesondere Immunglobuline oder andere Proteine, die eine Fc-Domäne aufweisen.

### STAND DER TECHNIK

Biomoleküle wie Proteine, Polynukleotide, Polysaccharide und dergleichen gewinnen als Medikamente, als Diagnostika, als Zusatzstoffe in Nahrungsmitteln, Waschmitteln und dergleichen, als Forschungsreagenzien und für viele weitere Anwendungen zunehmend an kommerzieller Bedeutung. Der Bedarf an solchen Biomolekülen lässt sich - z.B. im Falle von Proteinen - in aller Regel nicht mehr durch Isolierung der Moleküle aus natürlichen Quellen befriedigen, sondern erfordert den Einsatz biotechnologischer Produktionsmethoden.

Die biotechnologische Herstellung von Proteinen beginnt typischerweise mit der Klonierung eines DNS-Fragmentes in einen geeigneten Expressionsvektor. Nach Transfektion des Expressionsvektors in geeignete prokaryontische oder eukaryontische Expressionszellen und anschließender Selektion transfizierter Zellen werden letztere in Fermentern kultiviert und das gewünschte Protein zur Expression gebracht. Anschließend erfolgt die Ernte der Zellen bzw. des Kulturüberstandes und die Aufarbeitung und Reinigung des darin enthaltenen Proteins.

Im Falle eukaryontischer Expressionssysteme, also bei Verwendung von Säugetierzellkulturen wie etwa CHO- oder NS0-Zellen, konnte in den zurückliegenden 15 Jahren eine deutliche Steigerung der beim Expressionsschritt erreichbaren Konzentration des gewünschten Proteins in den Zellkulturen bzw. Zellkulturüberständen erzielt werden. Im gleichen Zeitraum stieg die Bindekapazität von Chromatographiematerialien, die bei der anschließenden Aufreinigung der Proteine eingesetzt werden, im Vergleich nur geringfügig. Aus diesem Grund besteht ein dringender Bedarf an verbesserten, optimierten Aufreinigungsverfahren für Biomoleküle, insbesondere Proteine, die in großem, industriellen Maßstab durchgeführt werden können.

Im Falle von Biopharmazeutika, wie etwa als Medikamente eingesetzten Proteinen, z.B. therapeutischen Antikörpern, ist neben der Produktausbeute auch die Abtrennung von Verunreinigungen von großer Bedeutung. Hierbei können prozess- und produktabhängige Verunreinigungen unterschieden werden. Die prozessabhängigen Verunreinigungen beinhalten Komponenten der Wirtszellen wie Proteine (Wirtszellproteine, "Host cell proteins", HCP) und Nukleinsäuren und stammen aus der Zellkultur (wie Medienbestandteile) oder aus der Aufarbeitung (wie etwa Salze oder abgelöste Chromatographie-Liganden). Produktabhängige Verunreinigungen sind molekulare Varianten des Produkts mit abweichenden Eigenschaften. Hierzu zählen verkürzte Formen wie Vorläufer und hydrolytische Abbauprodukte, aber auch modifizierte Formen, entstanden beispielsweise durch Desaminierungen, falsche Glykosylierungen oder falsch verknüpfte Disulfidbrücken. Ebenso zu den produktabhängigen Varianten zählen Polymere und Aggregate. Weitere Verunreinigungen sind Kontaminanten. Damit werden alle weiteren Materialien chemischer, biochemischer oder mikrobiologischer Natur bezeichnet, die nicht direkt zum Herstellungsprozess gehören. Kontaminanten sind z.B. Viren, die unerwünschter Weise in Zellkulturen auftreten können.

Verunreinigungen führen im Falle von Biopharmazeutika zu Sicherheitsbedenken. Diese werden verstärkt, wenn - wie sehr häufig bei Biopharmazeutika - die Verabreichung der therapeutischen Proteine über Injektion oder Infusion direkt in die Blutbahn erfolgt. So können Wirtszellkomponenten zu allergischen Reaktionen oder immunopathologischen Effekten führen. Daneben können Verunreinigungen auch zu einer unerwünschten Immunogenität des verabreichten Proteins führen, also eine unerwünschte Immunreaktion des Patienten auf das Therapeutikum auslösen, bis hin zu einem lebensbedrohenden anaphylaktischen Schock. Daher besteht ein Bedarf an geeigneten Reinigungsprozessen, mit denen alle unerwünschten Substanzen auf ein unbedenkliches Maß abgereichert werden können.

Andererseits können auch im Falle von Biopharmazeutika wirtschaftliche Aspekte nicht ignoriert werden. So sollen die eingesetzten Produktions- und Reinigungsverfahren die Rentabilität des damit erzeugten biopharmazeutischen Produkts nicht gefährden.

Als Aufarbeitungs-/ und Reinigungsschritt von Proteinen kommt neben Filtrations- und Fällungsschritten den (säulen-)chromatographischen Verfahren eine ganz zentrale Bedeutung zu. So beinhaltet häufig gerade die Antikörper Anreicherung einen affinitätschromatographischen Reinigungsschritt. Dementsprechend sind heutzutage zahlreiche säulenchromatographische Verfahren und hierbei verwendbare Chromatographiematerialen bekannt.

Affinitätschromatographie-Matrices werden bei der industriellen Reinigung von verschiedenen Substanzen als stationäre Phase eingesetzt. Über immobilisierte Liganden lassen sich Substanzen spezifisch anreichern und reinigen, die eine gewisse Affinität zu dem jeweils verwendeten Liganden besitzen. Für die industrielle Aufreinigung von Antikörpern (Immunglobulinen), insbesondere die Reinigung monoklonaler Antikörper, wird häufig immobilisiertes Protein A als initialer Reinigungsschritt verwendet. Protein A ist ein Protein mit etwa 41 kDA aus *Staphylococcus aureus,* das mit hoher Affinität (10⁻⁸ M - 10⁻¹² M an humanem IgG) an die CH₂/CH₃-Domäne der Fc-Region von Immunglobulinen bindet. Bei der Protein A-Chromatographie binden Immunglobuline oder Fusionsproteine, die eine Protein A-bindende Fc-Region aufweisen, aus der mobilen Phase spezifisch an den Protein A-Liganden, der kovalent an einen Träger (z.B. Sepharose) gekoppelt ist. Protein A aus *Staphylococcus aureus* (wildtyp Protein A) sowie gentechnisch verändertes, rekombinantes Protein A (rec. Protein A) interagiert über nicht-kovalente Wechselwirkungen mit der konstanten Region (Fc-Fragment) der Antikörper. Diese spezifische Interaktion kann ausgenutzt werden um Verunreinigungen effizient von dem Antikörper abzutrennen. Durch eine Veränderung des pH-Werts kann die Interaktion zwischen Antikörper und Protein A-Liganden gezielt aufgelöst und die Antikörper von der stationären Phase freigesetzt bzw. eluiert werden. Bottomley et al. (18) beschreibt Varianten der B Domäne von Protein A mit geringerer Affinität für IgG mit denen gebundene IgG Moleküle unter milderen Bedingungen eluiert werden können. WO 92/09633 A1 (19) offenbart Polypeptide mit 2 bis 4 IgG Bindedomänen, die von *Staphylococcus aureus* Protein A abgeleitet sind und durch eine geringere Affinität für IgG Immunglobuline gekennzeichnet sind.

Neben Protein A als Affinitätsligand sind derzeit viele weitere Moleküle bekannt, die an das Fc-Fragment binden. So werden auch einzelne Domänen des Protein A anstelle des kompletten Proteins verwendet (8). Hierbei sind gerade von der B-Domäne des Protein A verschiedene Protein-Varianten bekannt, die sich zur Bindung von Fc-Fragment-enthaltenden Molekülen eignen (16, 17). Diese unterschiedlichen Varianten unterscheiden sich im Wesentlichen durch Mutationen, die eingeführt wurden, um die Stabilität oder Bindungsaffinität zu steigern. Diese Mutanten der B-Domäne werden zumeist Z-Domäne oder Protein Z genannt. Neben Protein A oder Protein G eignen sich auch verschiedene Peptide für die selektive Bindung an das Fc-Fragment (14). Das derzeit große Interesse an Affinitätsliganden für das Fc-Fragment lässt hierbei vermuten, dass noch weitere Affinitätsliganden gefunden werden.

Die Affinitätschromatographie und insbesondere die häufig verwendete Protein A Chromatographie ist kostenaufwendig und gerade bei steigenden Produktkonzentrationen im Fermenter und hohen Produktmengen kommt es bei chromatographischen Reinigungsprozessen zu Limitierungen. Kritische Punkte sind hierbei: Beladungskapazitäten, Zyklenzahl, Prozesszeiten, Poolvolumina und Puffermengen. Alternative Reinigungsverfahren sind daher für die Zukunft unerlässlich. Eine generelle Übersicht zu konventionellen Reinigungsstrategien, wie auch der Affinitätschromatographie und alternative Methoden der Affinitätschromatographie bieten folgende Artikel (7, 10).

Eine neuere Methode der Affinitätschromatographie verwendet nicht einen stets immobilisierten Affinitätsliganden, sondern zunächst einen solubilisierten Affinitätsliganden, der mit dem Zielprotein gemischt wird (11). Der Affinitätsligand trägt hierbei einen Fusionstag oder ein Fusionsprotein, welches die im zweiten Schritt erfolgende Immobilisierung des Affinitätsliganden auf eine Feste Phase ermöglicht. Als nächster Schritt wird wie in der herkömmlichen Affinitätschromatographie unter geeigneten Bedingungen das Zielprotein vom Ligand getrennt und somit von der Säule eluiert.

Die hier beschriebene Erfindung bedient sich der Affinitätspräzipitation anstelle der Affinitätschromatographie zur Reinigung von Biomolekülen. Diese Methode scheint gerade im größeren Maßstab viel Potential zu haben (1, 2).

Affinitätspräzipitation ist die effektivste Art der Protein-Präzipitation (2). Die Präzipitation eines Proteins aus einer Lösung im Allgemeinen ist ein häufig angewendetes und bekanntes Verfahren. So wurden bereits viele Proteine über Ammoniumsulfat-Fällung aus einem Proteingemisch aus Lösung abgetrennt. Bei der Präzipitation werden Makromoleküle (z.B. Proteine) aus der Lösung in Partikel überführt. Dieser Schritt führt abhängig vom Dichteunterschied zwischen Partikel und Lösung und dem Partikeldurchmesser zur Präzipitation. Diese Präzipitation verläuft jedoch zumeist unspezifisch.

Zur selektiveren Anreicherung eines Moleküls z.B. Proteins entwickelte sich daher die Affinitätspräzipitation. Bei der Affinitätspräzipitation wird die selektive Bindung eines Affinitätsmoleküls an ein Zielmolekül ausgenutzt. Affinitätsmoleküle können hierbei z.B. Proteine, Peptide, Oligonukleotide oder auch kleine chemische Moleküle sein. Man unterscheidet bei der Affinitätspräzipitation zwei Prinzipien, die Affinitätspräzipitation erster und zweiter Ordnung (7). Bei der Affinitätspräzipitation erster Ordnung besitzt sowohl das Affinitätsmolekül als auch das Zielmolekül zwei Bindungsstellen, so dass eine Netzwerkausbildung zwischen beiden Molekülen möglich ist und sich ein Affinitätskomplex bildet, der bei einer bestimmten Größe sedimentiert. Bei der Affinitätspräzipitation zweiter Ordnung werden z.B. Affinitätsmakroliganden (AML) eingesetzt. Bei dieser Art der Präzipitation wird das Affinitätsmolekül an eine stimulierbare Substanz, zumeist an ein Polymer gebunden. Die stimulierbare Substanz ändert durch eine Veränderung der Umgebungsbedingungen, wie z.B. einer pH- oder Temperatur-Verschiebung sein Löslichkeitsverhalten, und es kommt zur Präzipitation. Anders als bei der Affinitätspräzipitation erster Ordnung wird hier kein bifunktionelles Affinitätsmolekül und auch kein bifunktionelles Zielmolekül benötigt.

Bei der zumeist angewendeten Affinitätspräzipitation werden derzeit die Affinitätsliganden an Polymere oder andere Mediatoren gekoppelt (15). Ljungquist et al. (20) offenbart rekombinante Protein Z Domänen von Protein A, welche mindestens einen zusätzlichen Cystein-Rest zur Kopplung an Thiopropylsepharose enthalten.
Die Affinitätspräzipitation von Fc-Fragment enthaltenden Molekülen ist bereits in einigen Arbeiten beschrieben wurden. Die derzeit häufigste Anwendung ist durch die Verwendung von so genannten "Smart-Polymers" gekennzeichnet. "Smart-polymers" (oder Stimuliresponsive "intelligent" polymers oder Affinitätsmakroliganden) sind Polymere die auf äußere Einflüsse (physische oder chemische Stimuli) ihre Eigenschaften ändern. Diese Stimuli können z.B. pH-Änderungen oder Temperatur-Änderungen sein (12-13). Zumeist reagieren "Smart-Polymere" auf Ihren Stimuli mit der Präzipitation in Lösung. Diese Präzipitation lässt sich nach gewünschter Abtrennung der überstehenden Lösung durch geeignete Bedingungen rückgängig machen. "Smart-Polymere" können mit verschiedenen Biomolekülen konjugiert werden, was zu einer großen Sammlung an Polymer-Biomolekül Systemen führt, die auch für verschiedenste Anwendungen eingesetzt werden können. Beispiele für diese Biomoleküle sind z.B. Proteine, Oligonukleotide und Zucker.

Eine weitere Art der Affnitätspräzipitation ist die kürzlich beschriebene "Affinity sinking" Methode. Bei dieser Art der Affinitätspräzipitation wird ein verlinkendes Molekülgerüst verwendet, um mehrere Affinitätsliganden miteinander zu verbinden. Dieses ermöglicht dann die benötigte Netzwerkausbildung für die Präzipitation. Die Bindung des Affinitätsliganden an den Netzwerkausbildner kann hierbei sowohl nicht-kovalent als auch kovalent erfolgen. Diese Methode wurde kürzlich in dem Patent "Compositions and methods for purifying and crystallizing molecules of interest" beschrieben (6). Hier wird zunächst eine Antikörper enthaltende Lösung mit einem Affinitätsliganden der mit einem Quervernetzer kovalent verbunden ist vermischt. Hierbei ist noch keine Präzipitation zu beobachten. Erst nach der nun folgenden Zugabe eines Koordinations-ions oder -moleküls kommt es zur Präzipitation. In einer zweiten ähnlichen Anwendung wird der Affinitätsligand mit einem Biotin-bindenden Protein verknüpft, der mit dem Mediator Avidin ein Netzwerk ausbildet (9).

In dem Patent US 7,083,943 wird eine Affinitätspräzipitation beschrieben, bei der eine Bindungsdomäne für das Zielprotein mit einer Gerüst ("Scaffold")-Domäne verknüpft wird, deren Aminosäuresequenz die Neigung zur Präzipitation erleichtern soll.

Chen und Hoffman (15) beschreiben die Affinitätspräzipitation von IgG mit poly(N-isopro-pylacrylamid)-Protein A-Konjugaten. Die Präzipitation von Antikörpern mit unmodifiziertem Protein A hat sich dagegen als nicht effektiv erwiesen.

Ein Nachteil der im Stand der Technik beschriebenen Methoden ist, dass bei der Affinitätspräzipitation erster Ordnung nur über das verlinkende Molekül eine Präzipitation möglich ist, bzw. dass bei der Affinitätspräzipitation zweiter Ordnung eine stimulierbare Substanz erforderlich ist. Weitere Nachteile der meisten bekannten Affinitätspräzipitationen sind zum einen a) die sterische Hinderung, welche durch die Bindung von hochmolekularen Affinitätsmakroliganden die Bindung des Zielproteins limitiert b) die Resolubilisierung des präzipitierten Polymers/ Komplexes ist langsam c) die nichtspezifische Mitfällung von Verunreinigungen, die zumeist einen zweiten Präzipitationsschritt erfordern d) der zusätzliche Schritt der Bindung eines Affinitätsproteins an das Polymer bzw. den Quervernetzer (2-5)

### KURZE ZUSAMMENFASSUNG DER ERFINDUNG

Die vorliegende Erfindung betrifft eine Affinitätspräzipitation mit Hilfe eines Bindungsproteins mit zwei Bindungsstellen, bei dem vollständig auf ein zusätzliches Fusionsprotein oder ein Linkermolekül verzichtet wird. Allein das Affinitätsprotein reicht zur Fällung aus, und eine stationäre Phase wird nicht benötigt. Die hier verwendete Erfindung erleichtert auch die Rückgewinnung des Affinitätsproteins.

Die Erfindung betrifft insbesondere ein Verfahren zur selektiven Anreicherung von Immunglobulinen oder anderen Proteinen, die eine Fc-Domäne enthalten (Zielprotein), mit den Schritten:
a. Bereitstellen einer Lösung, die das Zielprotein enthält;
b. Einbringen eines Fc-bindenden Proteins mit genau zwei Bindungsstellen unter Bedingungen, die eine Bindung ermöglichen;
c. Abtrennen des Präzipitates von der flüssigen Phase;
d. Lösen der Bindung des Zielproteins von dem Fc-bindenden Protein.

In einem weiteren Aspekt betrifft die Erfindung ein solches Verfahren, bei dem das Fc-bindende Protein ein Dimer einer Fc-bindenden Domäne von Protein A oder Protein G ist. Bevorzugt sind dabei die beiden Monomere des Dimers über eine Disulfidbrücke miteinander verknüpft.

In einem weiteren Aspekt betrifft die Erfindung ein solches Verfahren beidem das Dimer ein Homodimer ist, dessen Monomere die SEQ ID NO: 1, SEQ ID NO: 2, SEQ ID NO: 3, oder SEQ ID NO: 7 oder eine Sequenz haben, die in 1, 2, 3, 4, 5, 6, 7, 8, 9, 10, 11, 12, 13, 14, 15, 16, 17, 18, 19 oder 20 Aminosäuren von der SEQ ID NO: 1 abweicht.

In einem weiteren Aspekt wird das Fc-bindende Protein in einem Verhältnis von 0,5-20 gegenüber dem Zielprotein eingesetzt. Die Lösung in Schritt a. weist vorzugsweise einen pH-Wert von 5,5 - 8 auf. Vorzugsweise wird das Lösen der Bindung in Schritt d. bei einem pH-Wert von 2 - 4,5 erreicht.

In einem weiteren Aspekt betrifft die Erfindung ein Fc-bindendes Protein, das aus 2 identischen Untereinheiten besteht, die die Sequenz SEQ ID NO: 1, SEQ ID NO: 2, SEQ ID NO: 3, SEQ ID NO: 7 oder eine Sequenz haben, die in 1, 2, 3, 4, 5, 6, 7, 8, 9, 10, 11, 12, 13, 14, 15, 16, 17, 18, 19 oder 20 Aminosäuren von der SEQ ID NO: 1 abweicht, wobei die beiden Untereinheiten über eine kovalente Bindung miteinander verknüpft sind. Vorzugsweise ist die kovalente Bindung eine Disulfidbindung.

### KURZE BESCHREIBUNG DER ZEICHNUNGEN

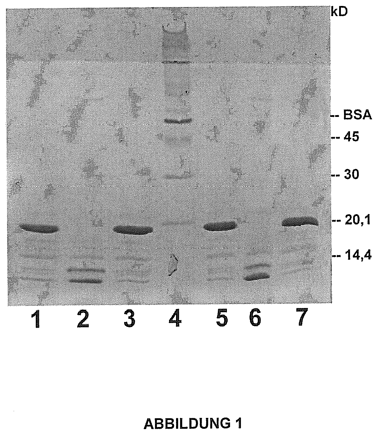

**Abbildung 1****:** SDS-PAGE des Z-Dimers. Zum Nachweis der Dimerisierung über Cysteine wurde zum einen lodacetamid oder lodacetamid mit Dithiothreitol vor dem Auftrag auf das SDS-PAGE zugegeben.

| **Spur** | **Probe** |
|---|---|
| **1** | rekombinantes Z-Dimer versetzt mit 0,6 µmol lodacetamid |
| **2** | rekombinantes Z-Dimer versetzt mit 0,3 µmol DTT und lodacetamid |
| **3** | reines rekombinantes Z-Dimer |
| **4** | Kombi Marker |
| **5** | rekombinantes Z-Dimer versetzt mit 0,2 µmol lodacetamid |
| **6** | rekombinantes Z-Dimer ver-setzt mit 0,1 µmol DTT und 0,2 µmol lodacetamid |
| **7** | reines rekombinantes Z-Dimer |

**Abbildung 2****:** Affinitätspräzipitation mittels Z-Dimer. Als erster Schritt wird Protein Z oxidiert. Im zweiten Schritt wird das Z-Dimer zu einer Antikörper (mAB) enthaltenen Lösung gegeben, welches zur selektiven Präzipitation des Antikörpers führt.
**Abbildung 3****:** UV-Diagramm der lonenaustauschchromatographie unter sauren Bedingungen zur Abtrennung des Z-Dimers vom Antikörper (Experiment 2). In Rot ist das UV-Chromatogramm bei 220 nm, in Blau das UV-Chromatogramm bei 280 nm und in Grün der steigende Gehalt an Puffer B (20 mM Phosphat, 3 M NaCl) gezeigt.
**Abbildung 4****:** SDS-PAGE zu Experiment 2.

| **Spur** | **Probe** |
|---|---|
| **1** | gereinigter Antikörper |
| **2** | Protein Z Dimer |
| **3** | Überstand nach der Präzipitation |
| **4** | Überstand nach einem optionalen Waschschritt |
| **5** | Resuspendiertes Pellet (Antikörper mit Z-Dimer) |
| **6** | Peak 1 der Ionenaustauschchromatographie bei saurem pH (Antikörper) |
| **7** | Peak 2 der sauren Ionenaustauschchromatographie bei saurem pH (Z-Dimer) |
| **8** | Kombi Marker |

### DETAILLIERTE BESCHREIBUNG DER ERFINDUNG

Die vorliegende Erfindung betrifft Verfahren zur Abreicherung von Verunreinigungen, insbesondere Wirtszellprotein (Host Cell Proteins, HCP) und DNS aus Proteinzusammensetzungen, wie sie aus Zellkulturen erhalten werden, in denen Proteine rekombinant oder endogen exprimiert werden. Insbesondere betrifft die Erfindung Verfahren zur Reinigung bzw. Anreicherung eines Proteins (Zielprotein) durch die Bindung eines Fc-bindenden Proteins oder Multimers von diesem mit mindestens zwei Bindungsstellen. In weiteren Schritten wird das Präzipitat abgetrennt und anschließend die Bindung des Fc-bindenden Proteins zum Zielprotein durch geeignete Bedingungen getrennt.

Die vorliegende Erfindung betrifft ein Verfahren zur selektiven Anreicherung von Immunglobulinen oder anderen Proteinen, die eine Fc-Domäne enthalten (Zielprotein), mit den Schritten:
a. Bereitstellen einer Lösung, die das Zielprotein enthält;
b. Einbringen einer eines Fc-bindenden Proteins mit genau zwei Bindungsstellen unter Bedingungen, die eine Bindung ermöglichen;
c. Abtrennen des Präzipitates von der flüssigen Phase;
d. Lösen der Bindung des Zielproteins von dem Fc-bindenden Protein.

Das Zielprotein kann insbesondere ein Immunglobulin oder ein Protein sein, das die Fc-Domäne eines Immunglobulins enthält und an Protein A oder Fragmente von Protein A binden kann. Immunoglobuline bestehen aus zwei schweren und zwei leichten Ketten. Die schweren Ketten haben jeweils eine variable und drei bis vier konstante Domänen abhängig vom Immunglobulin. Bezeichnet werden diese analog als VH und CH1, CH2, CH3. Die variablen Domänen einer leichten und einer schweren Kette bilden die Antigenbindungsstelle. Die Domäne CH2 enthält eine Kohlenhydratkette, die eine Bindungsstelle für das Komplementsystem bildet. Die CH3 Domäne enthält die Fc-Rezeptor-Bindungsstelle. Zielproteine, auf die das erfindungsgemässe Verfahren angewendet werden kann, sind alle Proteine die eine Fc-Domäne aufweisen. Beispiele für CH2/CH3 Regionen enthaltene Proteine sind Antikörper, Immunoadhesine und Fusionsproteine in denen das Protein von Interesse mit einer CH2/CH3 Region verbunden ist. In einer Darstellungsform der Erfindung ist das Zielprotein z.B. ein Antikörper der eine CH2/CH3 Region besitzt und somit zur Bindung an Protein A befähigt ist. Der Ausdruck CH2/CH3 Region bezieht sich auf die Aminosäuren in der Fc-Region eines Antikörpers, die mit Protein A interagieren.

Das Fc-bindende Protein weist erfindungsgemäß genau zwei Bindungsstellen für eine Fc-Domäne auf.

In einem weiteren Aspekt betrifft die Erfindung ein solches Verfahren, bei dem das Fc-bindende Protein ein Dimer einer Fc-bindenden Domäne von Protein A oder Protein G ist. Bevorzugt sind dabei die beiden Monomere des Dimers über eine Disulfidbrücke miteinander verknüpft.

Unter einem Fc-bindenden Protein sind dabei Proteine oder Peptide zu verstehen, die befähigt sind an die Fc-Region zu binden. Bevorzugt binden Fc-bindende Proteine mit einer Dissoziationskonstante (K_{D}-Wert) im Bereich von 10⁻²-10⁻¹³M.

In bevorzugten Ausführungsformen der Erfindung ist das Fc-bindende Protein ein Homo- oder Heterodimer von Fc-bindenden Domänen, die die in Tabelle 1 aufgeführten Sequenzen aufweisen oder enthalten:

| SEQ ID NO : | Sequenz | Beschreibung |
|---|---|---|
| 1 | | Z-Domäne-Cys-Tag |
| 2 | | Z-Domäne-Cys |
| 3 | | Z-Domäne |
| 4 | | E Domäne von Protein A (Swissprot P02967) |
| 5 | | D Domäne von Protein A (Swissprot P02967) |
| 6 | | C Domäne von Protein A (Swissprot P02967) |
| 7 | | B Domäne von Protein A (Swissprot P02967) |
| 8 | | A Domäne von Protein A (Swissprot P02967) |
| 9 | | Protein G Fc-bindende Domäne aus Streptococcus Sp. (Uniprot Q53337) |
| 10 | | Protein G Fc-bindende Domäne aus Streptococcus dysgalactiae (YP_002997067) |
| 11 | | IgG bindende Domäne aus Streptococcus equi (YP 002123072) |

Unter einem Homodimer ist in diesem Zusammenhang ein Fc-bindendes Protein zu verstehen, das aus zwei Untereinheiten gleicher Sequenz aufgebaut ist.

Unter einem Heterodimer ist in diesem Zusammenhang ein Fc-bindendes Protein zu verstehen, das aus zwei Untereinheiten verschiedener Sequenz aufgebaut ist, die jeweils eine Bindungsstelle für eine Fc-Domäne haben. Bevorzugt enthalten die Untereinheiten Sequenzen, die aus den Sequenzen der Tabelle 1 ausgewählt sind.

In einem weiteren Aspekt betrifft die Erfindung ein solches Verfahren, bei dem das Dimer ein Homodimer ist, dessen Monomere die SEQ ID NO: 1, SEQ ID NO: 2, SEQ ID NO: 3, SEQ ID NO: 7 oder eine Sequenz haben, die in 1, 2, 3, 4, 5, 6, 7, 8, 9, 10, 11, 12, 13, 14, 15, 16, 17, 18, 19 oder 20 Aminosäuren von der SEQ ID NO: 1 abweicht. Die Monomere, die in 1, 2, 3, 4, 5, 6, 7, 8, 9, 10, 11, 12, 13, 14, 15, 16, 17, 18, 19 oder 20 Aminosäuren von der SEQ ID NO: 1 abweichen, haben dabei die Eigenschaft, Fc-Domänen mit einem K_{D}-Wert im Bereich von 10⁻²-10⁻¹³M zu binden. Verfahren zur Bestimmung des K_{D}-Wertes sind dem Fachmann bekannt.

Bindungsbedingungen sind Bedingungen, bei denen eine Bindung an das Zielprotein durch die Fc-bindenden Proteine stattfindet, vorzugsweise in einem pH-Bereich von pH 5,5 - 9, bevorzugt 6 - 8.

Die Präzipitation erfolgt unter Bindungsbedingungen spontan, wie sie zum Beispiel in zell-freiem eukaryontischen Kulturüberstand vorliegen. Es besteht keine Notwendigkeit, die erfindungsgemäßen Dimere zur Förderung der Präzipitation mit Polymeren, z.B. Polyäthylenglykolen, zu verknüpfen.

In einem weiteren Aspekt wird das Fc-bindende Protein in einem molaren Verhältnis von 0,5 - 20 gegenüber dem Zielprotein eingesetzt.

Die Abtrennung des Präzipitates kann durch Zentrifugation und anschließender Abnahme des Überstandes, aber auch durch Filtrationstechniken erfolgen.

Das Lösen der Bindung zum Zielprotein erfolgt unter Bedingungen, die eine Trennung des Fc-bindenden Proteins vom Zielprotein erlauben. Vorzugsweise kann dies durch Einstellen eines pH-Bereiches zwischen pH 2 - 4,5 erreicht werden.

In einem weiteren Aspekt betrifft die Erfindung ein Fc-bindendes Protein, das aus 2 identischen Untereinheiten besteht, die die Sequenz SEQ ID NO: 1, SEQ ID NO: 2, SEQ ID NO: 3, SEQ ID NO: 7 oder eine Sequenz haben, die in 1, 2, 3, 4, 5, 6, 7, 8, 9, 10, 11, 12, 13, 14, 15, 16, 17, 18, 19 oder 20 Aminosäuren von der SEQ ID NO: 1 abweicht, wobei die beiden Untereinheiten über eine kovalente Bindung miteinander verknüpft sind. Vorzugsweise ist die kovalente Bindung eine Disulfidbindung.

### BEISPIELE

### Material und Methoden:

### Darstellung des dimerisierten Protein Z

Protein Z wurde als rekombinantes Protein aus E. coli erhalten. Die Abtrennung von Verunreingungen erfolgte nach der Abtrennung der Zelltrümmerüber eine lonenaustauschchromatographie.

### Proteinsequenz der verwendeten Z-Domäne:

Durch die Einbringung eines nicht nativen Cysteins in die Peptidkette der Z-Domäne ist es möglich, gezielt zwei Protein Z-Moleküle über eine Disulfidbrücke zu verknüpfen. Direkt nach der Reinigung des rekombinanten Protein Z liegt dieses durch Oxidation als dimerisiertes Protein vor.

### Zell-freier eukaryontischer Kulturüberstand

Der Zellkulturüberstand von auf sekretorische Produktion optimierte CHO-Zellen wurde nach mehreren Tagen Kultivation durch Filtration oder Zentrifugation erhalten.

### SEC-Analyse

Die Analyse von Protein-Verunreinigungen sowie des Antikörper-Gehaltes der Proben aus Experiment 1 erfolgte mittels analytischer Größenausschlusschromatographie (SEC). Es wurde eine TSK-GEL SW 3000 mit der vom Hersteller empfohlenen Vorsäule verwendet (TOSOH Bioscience). Die Analyse erfolgte an einer Dionex Ultimate Anlage mit Verfolgung des UV-Signals bei 280 und 220 nm.

### Ionenaustauschchromatographie zur Abtrennung des Z-Dimers vom Antikörper

Die lonenaustauschchromatographie erfolgte an einer ÄKTA Explorer 10 Anlage (GE Healthcare) mit Beobachtung der UV-Absorption bei 220 und 280 nm. Als Säulenmaterial wurde SP Sepharose FF verwendet (19 mL Gelbettvolumen). Nach dem Auftrag des bei saurem pH Wert resuspendierten Pellets wurde die Säule mit 20 mM Phosphatpuffer pH 3,3 equilibriert und anschließend der Antikörper sowie das Z-Dimer über einen 25 Säulenvolumen langen Gradienten voneinander getrennt. Zum Eluieren wurde ein 20 mM Phosphatpuffer mit 3 M NaCl bei pH 3,3 verwendet.

### Protein A HPLC

Zur Bestimmung des Antikörper-Gehaltes von Zellkulturfreiem Überstand sowie von gereinigtem Antiköper wurde eine Protein A HPLC an einer Waters oder Dionex-Anlage verwendet (Injektor Pumpen und Säulenofen W2790/5, UV-Detektor W2489). Der Antikörper-gehalt der Lösungen wurde mittels des UV-Signals des sauer eluierenden Peaks bestimmt.

### Anreicherung

Die bei den Versuchen erhaltenen Lösungen wurden teilweise für die analytischen Methoden ankonzentriert (Amicon Ultra, Ausschlussgröße 3 kD).

### SDS-PAGE:

Zur Überprüfung der einzelnen Fraktionen und Überstände wurde ein 20%iges homogenes SDS-Gel verwendet. Die Detektion der Proteinbanden erfolgte mittels Silberfärbung nach Heukeshoven.

### Wirtszellprotein-Analytik mittels ELISA-Assay

Im Experiment 3 wurde die Wirtszellprotein-Analytik mittels eines ELISA-Assays durchgeführt.

### DNA-Analytik

Die DNA-Analytik erfolgte nach Einzelstrangerzeugung durch eine enzymatisch katalysierte Nachweisreaktion.

### UF/DF

Für die UF/DF Versuche in Experiment 3 wurde eine 50 kDa Centramate Sheet PES (Polyethersulfon) der Firma Pall mit einer Fläche von 180 cm² verwendet. Die UF/DF wurde so gefahren, dass der Antikörper zunächst sechsfach ankonzentriert wurde und anschließend diafiltriert wurde (Austausch gegen 6 Volumen des Puffers), bevor 10 min bei vollständig geöffnetem Retentatventil zirkuliert wurde. Die Diafiltration wurde zwei mal wiederholt. Die Gesamte UF/DF wurde mit 50 mM Acetatpuffer + 100 mM Arginin + 150 mM NaCl pH 3,0 durchgeführt.

### Hydrophobe-Interaktions-Chromatographie (HIC)

Die Durchführung der Hydrophobe-Interaktions-Chromatographie (Experiment 3) erfolgte mit mit einer 27 mL Säule an einer ÄKTA-Anlage. Als Säulenmaterial wurde Toyopearl Phenyl 650 M von Tosoh verwendet. Hierzu wurde nach der UF/DF zum Retentat 3,5 M Ammoniumsulfatpuffer zugegeben bis eine Leitfähigkeit von 165 mS/cm erreicht war. Die mit Puffer (50 mM Acetat + 1,2 M Ammoniumsulfat pH 4) equilibrierte Säule wurde mit dem Retentat der UF/DF beladen und anschließend ein Gradient über 40 Bettvolumen zu den Puffer 50 mM Acetat pH 4 durchgeführt.

### Experimente:

### Nachweis der Dimerisierung des Protein Z über Cystein

Der Nachweis zur Dimerisierung des Protein Z über Cystein erfolgte mittels einer SDS-PAGE Analyse (Abbildung 1). Um sicher zu stellen, dass keine Oxidation des Protein Z durch die Aufarbeitung induziert wird, wurde zunächst mit lodacetamid die freien Cysteingruppen alkyliert. Zur Detektion des Monomers wurde zusätzlich mit Dithiothreitol (DTT) reduziert.
2,6 nmol Z-Dimer wurden mit DTT oder einem entsprechendem Volumen Puffer gemischt und fünf Minuten bei 95°C inkubiert. Anschließend wurde auf Raumtemperatur abgekühlt und lodacetamid oder ein entsprechendes Volumen Puffer zugegeben und weitere 20 min im Dunklen inkubiert. Nach fünf-minütiger Inkubation mit SDS-PAGE-Puffer wurde auf das SDS-PAGE aufgetragen.
Die SDS-PAGE-Analyse zeigt, dass durch Zugabe des Reduktionsmittel DTT das Z-Monomer gebildet wird und die Gel-Bande vom Z-Dimer verschwindet.

### Selektive Präzipitation mittels eines Z-Dimers

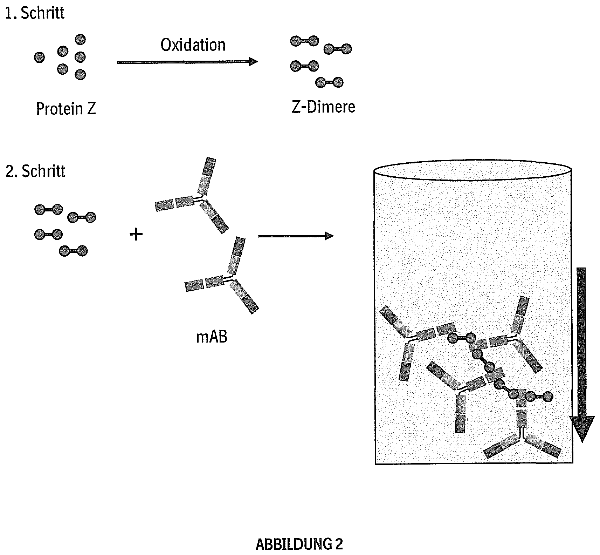

Die folgend aufgeführten Versuche zeigen, dass die selektive Präzipitation eines Antikörpers mit einem Z-Dimer erreicht werden kann (Abbildung 2, Experiment 1). Das Z-Dimer ist die über zwei nicht native Cysteine verknüpfte dimerisierte B-Domäne des Protein A's aus Staphylococcus aureus, welches eine Mutation gegenüber dem Wildtyp trägt (siehe Material und Methoden). Weiterhin kann in Experiment 2 gezeigt werden, dass das erhaltene Pellet durch Resuspension im sauren pH-Bereich wieder in Lösung gebracht werden kann und anschließend ein Antikörper durch eine lonenaustauschchromatographie bei saurem pH-Wert von dem Z-Dimer wieder abgetrennt werden kann.

### Experiment 1:

Es wurden unterschiedliche Verhältnisse von Protein Z zu Antikörper, welcher in Zell-freiem eukaryontischem Kulturüberstand vorlag, getestet. Weiterhin wurde das Volumen der einzelnen Ansätze variiert. In Versuch 1 wurden 0,13 µmol Z-Dimer mit 0,065 µmol IgG4 vorliegend in Zell-freiem eukaryontischem Kulturüberstand im Gesamtvolumen von 5,47 mL 2 h schüttelnd inkubiert. In Versuch 2 wurden 16,25 nmol Z-Dimer mit 16,25 nmol IgG1 vorliegend in Zell-freiem eukaryontischem Kulturüberstand im Gesamtvolumen von 5,2 mL ebenso 2 h schüttelnd inkubiert. Anschließend wurde 10 min bei 4000 rpm zentrifugiert und der Überstand vom Pellet getrennt. Der Antikörper-Gehalt des Überstandes wurde mittels des UV-Signals einer analytischen SEC sowie durch Protein A-Chromatographie bestimmt (Material und Methoden). Der Gehalt des Antikörpers im Pellet wurde durch Subtraktion des Antikörper-Gehaltes des Überstandes vom Gesamtgehalt des eingesetzten Antikörper erhalten. Hierbei konnte eine Präzipitation von bis zu 99% Antikörper beobachtet werden (Versuch 1: 99%/ Versuch 2: 76%). Weiterhin wurde der Kulturüberstand vor und nach der Präzipitation mittels analytischer SEC auf Protein-Verunreinigungen untersucht. Es wurde hierbei direkt das Chromatogramm vom Zell-freiem eukaryontischem Kulturüberstand mit dem Überstand nach der Präzipitation verglichen. Hierbei konnten Abreicherungen von Protein-Verunreinigungen im Bereich von 90-99% beobachtet werden (Versuch 1 90%/ Versuch 2 99%). Dass heißt, das 1% bzw. 10% der Protein-Verunreinigungen mitgefällt worden sind. Protein-Verunreinigungen können hierbei sowohl Wirtszellproteine als auch Fragmente oder Aggregate des Zielproteins sein.

### Experiment 2:

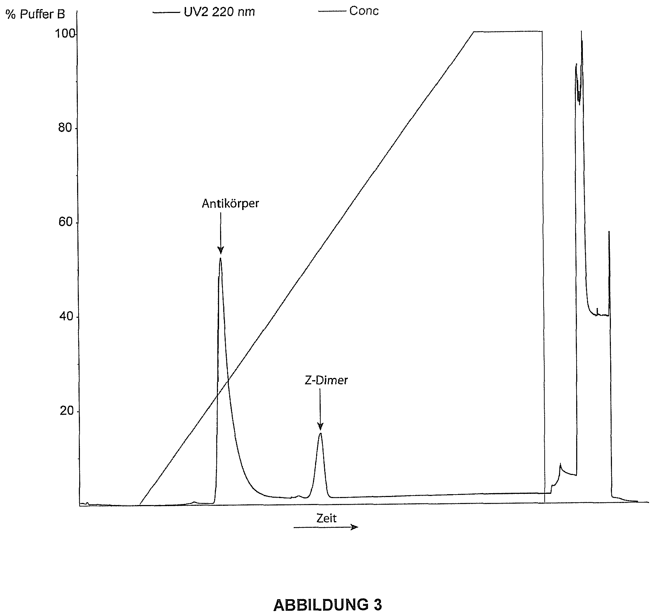

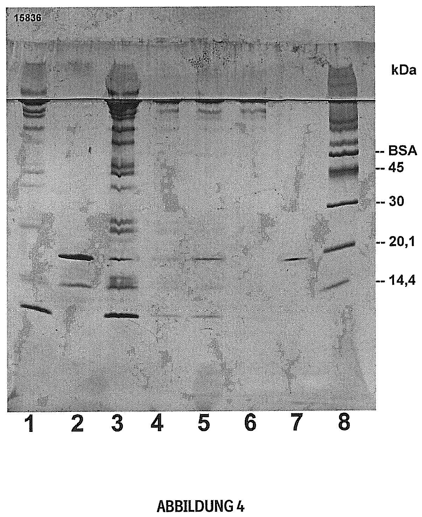

Zur Trennung des Z-Dimers vom Antikörper wurde ein Kationentauscher verwendet (SP Sepharose FF). 0,2 µmol gereinigter Antikörper wurden mit 0,2 µmol Z-Dimer in einem Volumen von 10,1 mL 36 min inkubiert. Nach anschließender Zentrifugation (4000 rpm, 10 min) wurde der Überstand abgenommen. Anschließend wurde das Pellet in 10 mL Phosphatpuffer pH 7,4 anteilig gelöst und erneut zentrifugiert (4000 rpm, 10 min). Das so erhaltene Pellet wurde in 20 mL Phosphatpuffer (20 mM Phosphat, pH 3,3) resuspendiert und über einen lonentauscher gereinigt. Durch einen Gradienten über 25 Bettvolumen konnte das Z-Dimer vom Antikörper abgetrennt werden (Abbildung 3 und 4).

### Experiment 3:

Es wurde ein mehrstufiger Antikörper-Reinigungsprozess mit der Affinitätspräzipitation als Capture Schritt durchgeführt.
Hierbei wurden 500 mg Antikörper (3,3 µmol) im Verhältnis 1:2 zu Z-Dimer (6,6 µmol) in der Präzipitation aus Zell-freiem Kulturüberstand eingesetzt. Nach ein-stündiger Inkubation unter leichtem Schütteln wurde die gesamte Suspension auf einen 0,22 µm Filter (Millipore) gegeben und der Überstand des Präzipitates so durch Filtration abgetrennt. Anschließend wurde das Präzipitat mit Puffer (231mL 50 mM Phosphatpuffer pH 7,4) gewaschen und im nächsten Schritt im Filter mit Acetatpuffer (179mL 50 mM Acetatpuffer + 100 mM Arginin + 150 mM NaCl pH 3,0) resuspendiert. Zur Abtrennung des Z-Dimers vom Antikörper wurde eine UF/ DF mit einer 50 kDa Membran durchgeführt. Als nächster Schritt diente eine HIC zur Abtrennung des Z-Dimers und weiteren Verunreinigungen.
Die Gesamtausbeute des Antikörpers lag nach der Präzipitation bei 98% und verringerte sich nach der UF/DF auf 87% und nach der HIC auf 64%. Neben den guten Ausbeuten bei der selektiven Präzipitation des Antikörpers konnte durch DNA und Wirtszellprotein Analytik gezeigt werden, dass die DNA durch den Präzipitationsschritt mit anschließender UF/DF um 99% und der Wirtszellproteingehalt um 99,9% gegenüber dem Ausgangswert (Zell-freier Kulturüberstand) reduziert werden konnten.

### Literatur

1) Labrou N, Clonis YD. (1994) J Biotechnol. 36(2), 95-119.
2) Hilbrig F, Freitag R. (2003) J Chromatogr B Analyt Technol Biomed Life Sci. 790(1-2),79-90.
3) Mattiasson B, Kumar A, Galaev IYu. (1998) J Mol Recognit. 11(1-6),211-6.
4) Vaidya AA, Lele BS, Kulkarni MG, Mashelkar RA. (1999) Biotechnol Bioeng. 64(4),418-25.
5) Garret-Flaudy F, Freitag R. (2000-2001) Biotechnol Bioeng. 71(3),223-34.
6) US 2006/ 0121519 A1 "Compositions and Methods for purifying and crystallizing molecules of interest"
7) H. Chmiel, Bioprozesstechnik, Spektrum Akademischer Verlag, 2 Auflage 2006
8) EP1179014B1
9) Patchornik, G. and Albeck A. (2005), Bioconjugate Chem. 16, 1310-1315.
10) Wilchek, M. and Gorecki, M. (1973), FEBS Letters. 31 , 1 , 149-152.
11)WO2009062942 KYHSE-ANDERSEN JAN [DK] "DUAL AFFINITY POLYPEPTIDES FOR PURIFICATION"
12) Hoffman, Controlled release (1987) 6, 297-305.
13) Hoffmann (1997) Intelligent polymers. In: Park K, ed. Controlled drug delivery. Washington: ACS Publications 485-98.
14) Haiou Yang, Patrick V. Gurgel und Ruben G. Carbonell (2009) Journal of Chromatography A 1216, 910-918.
15) Chen et Hoffman (1990), Biomaterials 11 (9), 631-634.
16) Nilsson et al 1987. B. Nilsson, T. Moks, B. Jansson, L. Abrahmsen, A. Elmblad, E. Holmgren, C. Henrichson, T.A. Jones and M. Uhlen, A synthetic IgG-binding domain based on staphylococcal protein A. Protein Eng. 1 (1987), pp. 107-113
17) Tashiro, M., Tejero, R., Zimmerman, D.E., Celda, B., Nilsson, B., and Montelione, G.T. 1997. High-resolution solution NMR structure of the Z domain of staphylococcal protein A. J. Mol. Biol. 272: 573-590.
18) Bottomley SP, Sutton BJ, Gore MG, Elution of human IgG from affinity columns containing immobilised variants of protein A., Jounal of Immunological Methods 182 (1995) 185-192)
19) WO 92/09633 A1 Gore et al. ,"IMMUNOGLOBULIN-BINDING PROTEINS AND RE-COMBINANT DNA MOLECULES CODING THEREFOR"
20) Ljungquist C, Jansson B, Moks T, Uhlen M., Thiol-directed immobilization of recombinant IgG-binding receptors, Eur J Biochem. 1989 Dec 22;186(3):557-61.

## Patentansprüche

1. Verfahren zur selektiven Anreicherung von Immunglobulinen oder anderen Proteinen, die eine Fc-Domäne enthalten (Zielprotein), mit den Schritten:
a. Bereitstellen einer Lösung, die das Zielprotein enthält; wobei die Lösung einen pH-Wert von 5,5 - 9, vorzugsweise 6 - 8 aufweist;
b. Einbringen eines Fc-bindenden Proteins mit genau zwei Bindungsstellen unter Bedingungen, die eine Bindung ermöglichen, wobei das Fc-bindende Protein ein Dimer einer Fc-bindenden Domäne von Protein A ist, wobei das Fc-bindende Protein nicht mit einem Polymer verknüpft ist;
c. Abtrennen des Präzipitates von der flüssigen Phase;
d. Lösen der Bindung des Zielproteins von dem Fc-bindenden Protein bei einem pH-Wert von 2-4,5.

2. Verfahren nach Anspruch 1, **dadurch gekennzeichnet, dass** die Fc-bindende Domäne ein Homodimer ist, dessen Monomere die Sequenzen SEQ ID NO: 1, SEQ ID NO: 2, SEQ ID NO: 3, SEQ ID NO: 7 oder eine Sequenz haben, die in 1, 2, 3, 4, 5, 6, 7, 8, 9, 10, 11, 12, 13, 14, 15, 16, 17, 18, 19 oder 20 Aminosäuren von der SEQ ID NO: 1 abweicht.

3. Verfahren nach Anspruch 1 oder 2, **dadurch gekennzeichnet, dass** die beiden Monomere des Dimers über eine Disulfidbrücke miteinander verknüpft sind.

4. Verfahren nach einem der vorangehenden Ansprüche, **dadurch gekennzeichnet, dass** das Fc-bindende Protein in einem molaren Verhältnis von 0,5-20 gegenüber dem Zielprotein eingesetzt wird.

## Claims

1. Process for the selective concentration of immunoglobulins or other proteins that contain an Fc domain (target protein), comprising the following steps:
a. preparing a solution that contains the target protein, wherein the solution has a pH of 5.5-9, preferably 6-8;
b. incorporating an Fc-binding protein with precisely two binding sites under conditions that allow binding to occur, wherein the Fc-binding protein is a dimer of an Fc-binding domain of protein A, wherein the Fc-binding protein is not linked to a polymer;
c. separating the precipitate from the liquid phase;
d. undoing the binding of the target protein from the Fc-binding protein at a pH of 2-4.5.

2. Process according to claim 1, **characterised in that** the Fc-binding domain is a homodimer, the monomers of which have the sequences SEQ ID NO: 1, SEQ ID NO: 2, SEQ ID NO: 3, SEQ ID NO: 7, or a sequence that differs from SEQ ID NO: 1 in 1, 2, 3, 4, 5, 6, 7, 8, 9, 10, 11, 12, 13, 14, 15, 16, 17, 18, 19 or 20 amino acids.

3. Process according to either claim 1 or claim 2, **characterised in that** the two monomers of the dimer are linked together by a disulphide bridge.

4. Process according to any of the preceding claims, **characterised in that** the Fc-binding protein is used in a molar ratio of 0.5-20 in relation to the target protein.

## Revendications

1. Procédé d'enrichissement sélectif d'immunoglobulines ou d'autres protéines, qui contiennent un domaine Fc (protéine cible), avec les étapes :
a. de mise à disposition d'une solution, qui contient la protéine cible ; ladite solution présentant un pH de 5,5 à 9, de préférence de 6 à 8 ;
b. d'introduction d'une protéine se liant à Fc avec précisément deux sites de liaison dans des conditions permettant une liaison, ladite protéine se liant à Fc étant un dimère d'un domaine se liant à Fc de la protéine A, ladite protéine se liant à Fc n'étant pas combinée à un polymère ;
c. de séparation du précipité de la phase liquide ;
d. de clivage de la liaison entre la protéine cible et la protéine se liant à Fc, à un pH de 2 à 4,5.

2. Procédé selon la revendication 1, **caractérisé en ce que** le domaine se liant à Fc est un homodimère, dont les monomères ont les séquences SEQ ID NO : 1, SEQ ID NO : 2, SEQ ID NO : 3, SEQ ID NO : 7 ou une séquence qui diverge de la SEQ ID NO : 1 par 1, 2, 3, 4, 5, 6, 7, 8, 9, 10, 11, 12, 13, 14, 15, 16, 17, 18, 19 ou 20 acides aminés.

3. Procédé selon la revendication 1 ou 2, **caractérisé en ce que** les deux monomères du dimère sont combinés l'un à l'autre par l'intermédiaire d'un pont disulfure.

4. Procédé selon l'une quelconque des revendications précédentes, **caractérisé en ce que** la protéine se liant à Fc est employée selon un rapport molaire de 0,5 à 20 par rapport à la protéine cible.


### SEQUENCE LISTING

<110> Boehringer Ingelheim International GmbH
<120> SELEKTIVE ANREICHERUNG VON ANTIKÖRPERN
<130> P01-2592/WO/1
<150> EP10155647.0
   <151> 2010-03-05
<160> 11
<170> PatentIn version 3.3
<210> 1
   <211> 72
   <212> PRT
   <213> Staphylococcus aureus
<400> 1
   Met Val Asp Asn Lys Phe Asn Lys Glu Gln Gln Asn Ala Phe Tyr Glu
   1               5                   10                  15
   Ile Leu His Leu Pro Asn Leu Asn Glu Glu Gln Arg Asn Ala Phe Ile
               20                  25                  30
   Gln Ser Leu Lys Asp Asp Pro Ser Gln Ser Ala Asn Leu Leu Ala Glu
           35                  40                  45
   Ala Lys Lys Leu Asn Asp Ala Gln Ala Pro Lys Ser Ser Ala Cys Arg
       50                  55                  60
   Arg Arg Arg Arg Arg Arg Arg Pro
   65                  70
<210> 2
   <211> 63
   <212> PRT
   <213> Staphylococcus aureus
<400> 2
   Met Val Asp Asn Lys Phe Asn Lys Glu Gln Gln Asn Ala Phe Tyr Glu
   1               5                   10                  15
   Ile Leu His Leu Pro Asn Leu Asn Glu Glu Gln Arg Asn Ala Phe Ile
               20                  25                  30
   Gln Ser Leu Lys Asp Asp Pro Ser Gln Ser Ala Asn Leu Leu Ala Glu
           35                  40                  45
   Ala Lys Lys Leu Asn Asp Ala Gln Ala Pro Lys Ser Ser Ala Cys
       50                  55                  60
<210> 3
   <211> 57
   <212> PRT
   <213> Staphylococcus aureus
<400> 3
   Asp Asn Lys Phe Asn Lys Glu Gln Gln Asn Ala Phe Tyr Glu Ile Leu
   1               5                   10                  15
   His Leu Pro Asn Leu Asn Glu Glu Gln Arg Asn Ala Phe Ile Gln Ser
               20                  25                  30
   Leu Lys Asp Asp Pro Ser Gln Ser Ala Asn Leu Leu Ala Glu Ala Lys
           35                  40                  45
   Lys Leu Asn Asp Ala Gln Ala Pro Lys
       50                  55
<210> 4
   <211> 50
   <212> PRT
   <213> Staphylococcus aureus
<400> 4
   Gln Gln Asn Ala Phe Tyr Gln Val Leu Asn Met Pro Asn Leu Asn Ala
   1               5                   10                  15
   Asp Gln Arg Asn Gly Phe Ile Gln Ser Leu Lys Asp Asp Pro Ser Gln
               20                  25                  30
   Ser Ala Asn Val Leu Gly Glu Ala Gln Lys Leu Asn Asp Ser Gln Ala
           35                  40                  45
   Pro Lys
       50
<210> 5
   <211> 57
   <212> PRT
   <213> Staphylococcus aureus
<400> 5
   Gln Asn Asn Phe Asn Lys Asp Gln Gln Ser Ala Phe Tyr Glu Ile Leu
   1               5                   10                  15
   Asn Met Pro Asn Leu Asn Glu Ala Gln Arg Asn Gly Phe Ile Gln Ser
               20                  25                  30
   Leu Lys Asp Asp Pro Ser Gln Ser Thr Asn Val Leu Gly Glu Ala Lys
           35                  40                  45
   Lys Leu Asn Glu Ser Gln Ala Pro Lys
       50                  55
<210> 6
   <211> 57
   <212> PRT
   <213> Staphylococcus aureus
<400> 6
   Asp Asn Asn Phe Asn Lys Glu Gln Gln Asn Ala Phe Tyr Glu Ile Leu
   1               5                   10                  15
   Asn Met Pro Asn Leu Asn Glu Glu Gln Arg Asn Gly Phe Ile Gln Ser
               20                  25                  30
   Leu Lys Asp Asp Pro Ser Gln Ser Ala Asn Leu Leu Ser Glu Ala Lys
           35                  40                  45
   Lys Leu Asn Glu Ser Gln Ala Pro Lys
       50                  55
<210> 7
   <211> 57
   <212> PRT
   <213> Staphylococcus aureus
<400> 7
   Asp Asn Lys Phe Asn Lys Glu Gln Gln Asn Ala Phe Tyr Glu Ile Leu
   1               5                   10                  15
   His Leu Pro Asn Leu Asn Glu Glu Gln Arg Asn Gly Phe Ile Gln Ser
               20                  25                  30
   Leu Lys Asp Asp Pro Ser Gln Ser Ala Asn Leu Leu Ala Glu Ala Lys
           35                  40                  45
   Lys Leu Asn Asp Ala Gln Ala Pro Lys
       50                  55
<210> 8
   <211> 57
   <212> PRT
   <213> Staphylococcus aureus
<400> 8
   Asp Asn Lys Phe Asn Lys Glu Gln Gln Asn Ala Phe Tyr Glu Ile Leu
   1               5                   10                  15
   His Leu Pro Asn Leu Thr Glu Glu Gln Arg Asn Gly Phe Ile Gln Ser
               20                  25                  30
   Leu Lys Asp Asp Pro Ser Val Ser Lys Glu Ile Leu Ala Glu Ala Lys
           35                  40                  45
   Lys Leu Asn Asp Ala Gln Ala Pro Lys
       50                  55
<210> 9
   <211> 55
   <212> PRT
   <213> Streptococcus Sp.
<400> 9
   Thr Thr Tyr Lys Leu Val Ile Asn Gly Lys Thr Leu Lys Gly Glu Thr
   1               5                   10                  15
   Thr Thr Lys Thr Val Asp Ala Glu Thr Ala Glu Lys Ala Phe Lys Gln
               20                  25                  30
   Tyr Ala Asn Asp Asn Gly Val Asp Gly Val Trp Thr Tyr Asp Asp Ala
           35                  40                  45
   Thr Lys Thr Phe Thr Val Thr
       50                  55
<210> 10
   <211> 55
   <212> PRT
   <213> Streptococcus dysgalactiae
<400> 10
   Thr Thr Tyr Lys Leu Val Ile Asn Gly Lys Thr Leu Lys Gly Glu Thr
   1               5                   10                  15
   Thr Thr Lys Ala Val Asp Ala Glu Thr Ala Glu Lys Ala Phe Lys Gln
               20                  25                  30
   Tyr Ala Asn Asp Asn Gly Val Asp Gly Val Trp Thr Tyr Asp Asp Ala
           35                  40                  45
   Thr Lys Thr Phe Thr Val Thr
       50                  55
<210> 11
   <211> 56
   <212> PRT
   <213> Streptococcus equi
<400> 11
   Thr Thr Tyr Arg Leu Val Ile Lys Gly Val Thr Phe Ser Gly Glu Thr
   1               5                   10                  15
   Ala Thr Lys Ala Val Asp Ala Ala Thr Ala Glu Gln Thr Phe Arg Gln
               20                  25                  30
   Tyr Ala Asn Asp Asn Gly Ile Thr Gly Glu Trp Ala Tyr Asp Thr Ala
           35                  40                  45
   Thr Lys Thr Phe Thr Val Thr Glu
       50                  55